# EUROPEAN PATENT SPECIFICATION

(11) **EP 1 774 984 B1**
(45) Date of publication and mention of the grant of the patent: **30.03.2011**
(21) Application number: 06255222.9
(22) Date of filing: 11.10.2006
(51) Int. Cl.: A61M 15/00

(54) **Gravity-actuated locking mechanism for drug container**
Schwerkraftbetriebener Verriegelungsmechanismus für einen Medikamentenbehälter
Mécanisme de verrouillage actionné par force de gravité pour un réservoir de médicament

(30) Priority: 11.10.2005 GB 0520645
(43) Date of publication of application: 18.04.2007
(73) Proprietor: Jagotec AG, 4132 Muttenz (CH)
(72) Inventor: Eggimann, Thomas, 4416 Bubendorf (CH); Gyimóthy, Gábor, 8125 Zollikerberg ZH (CH)
(74) Representative: Crump, Julian Richard John

(56) References cited:
- US-B1- 6 182 655

## Description

The present invention is concerned with a container for holding and dispensing a drug substance, in particular an inhaler device for dosing medicaments to be inhaled by a patient, containing a gravity-actuated locking mechanism for same, which permits opening of the container when it is held in a certain orientation but not in others.

US patent 6,182,655 describes a dry powder inhaler device for the administration of a pharmacological dry powder. The device consists of a housing, which contains a reservoir holding a dry powder formulation and having an exit port through which the formulation can be released, and a dosing mechanism comprising a dosing chamber adapted to receive a unit dose of the powder from the reservoir, which is moveable within the housing to transport a unit dose of powder formulation from a position directly under the reservoir exit port to a position proximate to a mouth-piece where it can be aerosolized upon actuation of the device by a user. Fitted over the mouth-piece is a cap that is connected to the housing. The cap covers the mouth-piece when the device is not in use. Connecting means between cap and dosing mechanism translates movement of the cap to the dosing mechanism, such that when the cap is opened to expose the mouth-piece, the dosing mechanism moves a unit dose in the dosing chamber in the manner described above. In this way, removal of the cap effectively loads the device in readiness for actuation by a user.

The device relies on gravity to feed the powder from the reservoir into the dosing chamber. To ensure that a unit dose is correctly and completely delivered to the dosing chamber, the device should be held in an orientation such that the reservoir exit port sits vertically, or substantially vertically, above the dosing chamber. In order to prevent a user readying the device for actuation with no dose or an incomplete dose in the dosing chamber, the device is equipped with a gravity-actuated locking mechanism for the cap. Essentially this locking mechanism only permits removal of the cap when the device is held by a user in an orientation such that the reservoir exit port is positioned vertically, or substantially vertically, above the dosing chamber. In the preferred embodiment described in the aforementioned patent, the reservoir exit port is held in the correct orientation with respect to the dosing chamber when the device is held such that both the housing and cap are held in the same, or substantially the same, horizontal plane. In such an orientation the cap can be removed and the device can be actuated to deliver correctly a unit dose. However, if the device is tilted or rotated out of this plane, then the gravity-actuated locking mechanism will prevent the cap being removed.

The gravity-actuated locking mechanism is provided in the form of two cooperating locking elements, which contact each other in locking engagement if it is attempted to remove the cap when the device is in an incorrect orientation as described above.

The first locking element is provided on a sledge that is connected to the cap and moves in conformity with the cap inside the housing along guide rails provided on the housing. The sledge also supports the dosing mechanism and translates movement of the cap to the dosing mechanism in the manner described above. The first locking element consists of abutment portions that extend downwards from the sledge. The sledge is adapted to move laterally within the housing over the second cooperating locking element. When the device is in the correct orientation for dosing, the abutment portions are adapted to move across the second locking element without being impeded by the it. However, if the device is tilted or rotated out of the correct orientation, the second locking element is urged under gravity to contact the first locking element in locking engagement if a user attempts to remove the cap.

In the specific embodiment described in the aforementioned patent, the second locking element consists of balls or spheres. Each ball is accommodated in, and is moveable about, a profile formed on the internal surface of the housing. Movement of a ball on such housing profile is gravity-actuated and is in response to any tilting or rotating movement of the device by a user. A housing profile is in the form of a socket comprising a substantially flat surface and a ramp that moves upwards and away from the substantially flat surface in the direction of the side wall of the housing. The substantially flat surface serves as a resting or home position occupied by a ball when the device is held in its correct dosing orientation described above. Each housing profile is bounded by two walls that are each directed outward of the resting position at an angle. The angled walls extend in the direction of the housing wall but do not extend all the way to the wall, leaving a narrow gap or channel between the end of each angled wall and the housing wall. The abutment channels (when the balls are in their home or resting positions) in response to the sledge being moved over the housing profiles.

When the device is in its correct dosing orientation, the balls sit in their respective home positions and are retained by the upward sloping ramp and the angled walls. With the balls so positioned, the cap can be opened, as the sledge moves laterally over the base profile in response to movement of the cap, and the abutment portions pass through the channels unimpeded.

However, if the device is tilted or rotated substantially out of its correct orientation in the manner described above, the ball will move under the influence of gravity away from its home position on the housing profile and roll across the ramp to cross the path of the channel such that if it is attempted to open the cap, the abutment portions of the sledge will contact the ball in locking engagement and prevent the cap from being opened.

The general principles of the device are fully described in the abovementioned patent US 6,182,655. In the remainder of this document elements of the device will be discussed only insofar as is necessary for the understanding of the orientation-dependant locking mechanism, which is the subject of this invention.

Aside from possessing all the necessary functional requirements, any commercially successful inhaler device should preferably be formed of low cost raw materials and must be capable of being massed produced efficiently and cheaply. Typically, in keeping with these commercial expedients, all of the component parts of a device are made of plastics materials that can be easily and cheaply formed by moulding and extrusion techniques. In keeping with this conventional approach, in the early development of the device described in the aforementioned patent, all parts were made of plastics materials. The choice of material forming the balls was considered to be of particular importance. The movement of the balls on the housing profile and the mechanical stresses resulting from the repeated impact with the abutment portions on the sledge called for a particularly hard-wearing and resilient material. The material selected was polyoxymethylene (POM).

During the early development activities of the device it was observed that the locking mechanism intermittently malfunctioned on some devices. On some occasions when a device was tilted or rotated out of the correct orientation the cap could still be removed, whereas on other occasions the cap could be opened irrespective of the device's orientation. It was felt that a malfunctioning of the locking mechanism could result in the cap being opened in an inappropriate orientation and a patient receiving an incorrect dose of drug.

Accordingly, there remains a need to provide an improved dry powder inhaler device that is provided with a locking mechanism that would reliably permit the cap to be opened by a patient when the device is in a correct dosing orientation.

In a first aspect of the present invention therefore there is provided a container for dispensing an active agent as defined in the apended claim 1.

Although the container may be configured such that any orientation can be set as the dosing orientation, i.e. that orientation wherein the cap is its unlocked state, in general the delivery orientation will be that in which the container is essentially horizontal; that is, when the housing and the cap are in the same horizontal, or essentially horizontal, plane.

In one example there is provided a container for dispensing an active agent, which container comprises a housing containing said active agent, a cap connected to the housing and gravity-actuated locking means for locking together the housing and the cap, said cap being unlocked state when the container is held in an orientation such that both the housing and the cap are in the same horizontal plane, but when tilted or rotated out of said plane said locking means are urged by gravity into locking engagement to lock the cap; wherein said locking means comprise a first locking element that is connected to the cap and is moveable in conformity with the cap inside the housing and a second locking element that is moveable under force of gravity on a profile formed on the internal surface of the housing in response to any tilting or rotating movement of the container out of the plane to engage the first locking element in locking engagement; characterised in that said second locking element is formed of a material that permits the second locking element to move freely under gravity on the housing profile even in the presence of an electrostatic field.

In another example there is provided an inhaler device for dispensing a dose of active agent to the respiratory tract of a patient, which device comprises a housing containing said active agent, a cap connected to the housing and gravity-actuated locking means for locking together the housing and the cap, said cap being in an unlocked state when the container is held in a first orientation, but when tilted or rotated out of this orientation said locking means are urged by gravity into locking engagement to lock the cap; wherein said locking means comprise a first locking element that is connected to the cap and is moveable in conformity with the cap inside the housing and a second locking element that is moveable under force of gravity on a profile formed on the internal surface of the housing in response to any tilting or rotating movement of the device out of the first orientation to engage the first locking element in locking engagement; characterised in that said second locking element is formed of a material that permits the second locking element to move freely under gravity on the housing profile even in the presence of an electrostatic field.

Yet another example shows an inhaler device for dispensing a dose of active agent to the respiratory tract of a patient, which device comprises a housing containing the active agent, a cap connected to the housing and gravity-actuated locking means for locking together the housing and the cap, said cap being in an unlocked state when the container is held in an orientation such that both the housing and the cap are in the same horizontal plane, but when tilted or rotated out of said plane said locking means are urged by gravity into locking engagement to lock the cap; wherein said locking means comprise a first locking element that is connected to the cap and is moveable in conformity with the cap inside the housing and a second locking element that is moveable under force of gravity on a profile formed on the internal surface of the housing in response to any tilting or rotating movement of the device out of the plane to engage said first locking element in locking engagement; characterised in that said second locking element is formed of a material that permits the second locking element to move freely under gravity on the housing profile even in the presence of an electrostatic field.

In still another example there is provided an inhaler device for dispensing a dose of active agent to the respiratory tract of the patient, which device comprises:
a housing having upper and lower parts, an inner surface of the lower part having a housing profile formed thereon;
a mouth-piece attached or adapted to be attached to said housing;
a cap connected to said housing and covering said mouth-piece, said housing containing:
   a reservoir for storing an active agent having an exit port through which active agent can be released;
   a dosing chamber for receiving a dose of active agent from the reservoir exit port; and
   means for delivering the dose from the dosing chamber to a patient via the mouth-piece on a stream of gas;
and gravity-actuated locking means for locking together the housing and the cap, the cap being in an unlocked state when the device is held in a first orientation, but when tilted or rotated out of said orientation said locking means are urged by gravity into locking engagement to lock the cap, said locking means comprising:
   a first locking element that is connected to the cap and moveable in conformity with the cap inside the housing; and
   a second locking element located on the housing profile and moveable on it from a first position to a second position under force of gravity in response to tilting or rotating movement of the device out of the first orientation to engage the first locking element in locking engagement;
characterised in that said second locking element is formed of a material that enables it to move freely under gravity on the housing profile even in an electrostatic field.

Further example shows an inhaler device for dispensing a dose of active agent to the respiratory tract of the patient, which device comprises:-
a housing having upper and lower parts, an inner surface of said lower part having a housing profile formed thereon;
a mouth-piece attached or adapted to be attached to said housing;
a cap connected to the housing and covering said mouth-piece, said housing containing:
   a reservoir for storing an active agent having an exit port through which active agent can be released;
   a dosing chamber for receiving a dose of active agent from the reservoir; and
   means for delivering the dose from the chamber to a patient via the mouth-piece on a stream of gas;
and gravity-actuated locking means for locking together the housing and the cap, said cap being in an unlocked state when the device is held in an orientation such that both the housing and the cap are in the same horizontal plane, but when tilted or rotated out of said plane said locking means are urged by gravity into locking engagement to lock the cap, said locking means comprising:
   a first locking element that is connected to the cap and moveable in conformity with the cap inside the housing, and
   a second locking element located on the housing profile and moveable on it from a first position to a second position under force of gravity in response to tilting or rotating movement of the device out of the plane to engage the first locking element in locking engagement;
characterised in that said second locking element is formed of a material that enables it to move freely under gravity on the housing profile even in an electrostatic field.

In yet another example there is provided an inhaler device for dispensing a dose of active agent to the respiratory tract of the patient, which device comprises:
a housing having upper and lower parts, an inner surface of said lower part having a housing profile formed thereon;
a mouth-piece attached or adapted to be attached to said housing;
a cap connected to the housing and covering said mouth-piece, said housing containing:
   a reservoir for storing an active agent having an exit port through which active agent can be released;
   a dosing chamber for receiving a dose of active agent from the reservoir; and
   means for delivering the dose from the chamber to a patient via the mouth-piece on a stream of gas;
and gravity-actuated locking means for locking together the housing and the cap, said cap being unlocked state when the device is held in an orientation whereby the reservoir exit port is positioned vertically, or substantially vertically, over the dosing chamber, but when tilted or rotated out of said orientation said locking means are urged by gravity into locking engagement to lock the cap, said locking means comprising:
   a first locking element that is connected to the cap and moveable in conformity with the cap inside the housing, and
   a second locking element located on the housing profile and moveable on it from a first position to a second position under force of gravity in response to any tilting or rotating movement of the device out of the aforementioned orientation to engage the first locking element in locking engagement;
characterised in that said second locking element is formed of a material that enables it to move freely under gravity on the housing profile even in the presence of an electrostatic field.

In a most preferred aspect of the present invention there is provided an inhaler device as described in US patent 6,182,655, characterised in that said locking balls are formed of material, or formed substantially of material that is able to move freely on or in the housing profile even in an electrostatic field.

After considerable investigation into the intermittent malfunction of the locking mechanism of the inhaler device of US 6,182,655, the present inventors found that during manufacture and subsequent handling, the device or component parts of the device, being made of plastics materials, would become electrostatically charged. The balls used in the locking device, being made of low density plastics materials, would intermittently become stuck in their home positions or they would be otherwise unable to move because they would stick to other adjacent parts of the device. As a result, the balls were unable to move, or did not move freely when the device was tilted or rotated, resulting in the malfunctioning of the lock.

In accordance with the present invention, the aforementioned problems associated with the device of US 6,182,655 have been alleviated by forming the second locking element of a material or materials that permits the second locking element to move freely on the housing profile in response to movement of the device even in the presence of an electrostatic field.

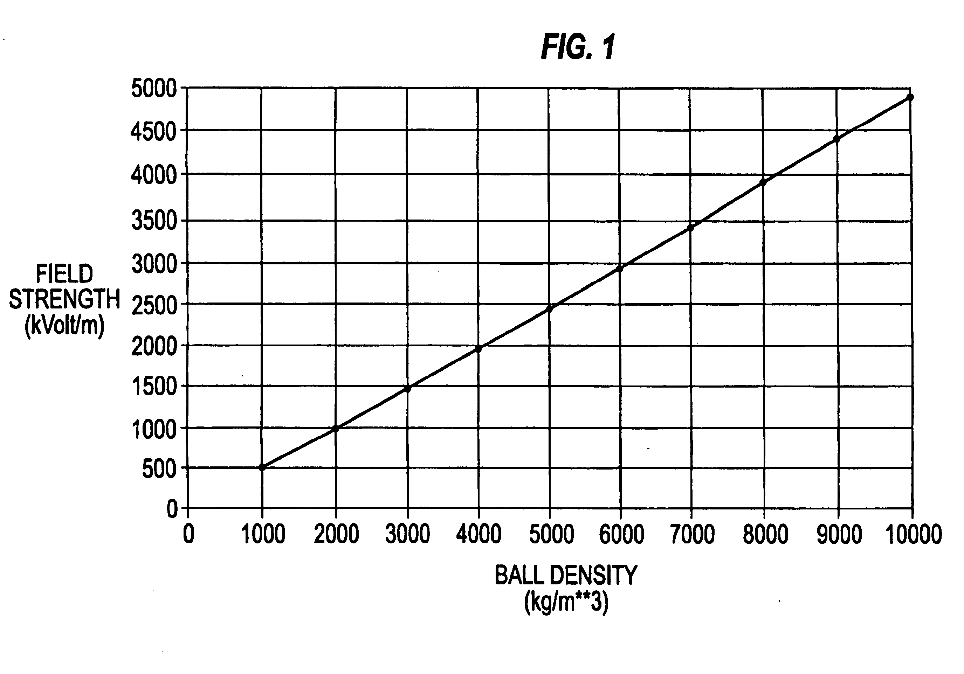

Whilst investigating the reasons for the malfunction of the gravity-actuated locking mechanism of the inhaler device of US 6,182,655, the inventors looked at the correlation between the density of the second locking element and its propensity to stick to adjacent components of the container when disposed in electrostatic fields of varying strengths. A substantially linear correlation was found between the field strength and the density of the second locking element needed to move freely on or within the housing profile within an electrostatic field. This relationship is shown in accompanying Figure 1.

The inventors found that electrostatic fields encountered as a result of manufacture and normal handling of the device are usually in the range of about 500 kVolt/m, although fields in the order of about 800kVolt/m can be experienced. Therefore, the second locking element is formed of a material having a density of at least about 1500 kg/m³, e.g. from about 1500 to about 10000, more particularly about 1500 to about 8000, in order that it is able to move freely under gravity in electric fields of this magnitude, or even higher magnitude should they be encountered.

Suitable materials for use in forming the second locking element include relatively high density plastics materials such as: Teflon®, polyurethane polymers, silicone polymers, polyethylene polymers, polypropylene polymers, epoxide resins; metals such as steel or aluminium; glass or ceramics; or elastomeric materials such as natural rubber and nitrylbutadiene rubbers. POM has a density of about 1400kg/m³ and as such is unsuitable as the sole material forming the second locking element. Similarly, acrylonitrile butadiene styrene polymers (ABS) are of low density and are not suitable as the sole material forming the second locking element.

Said second locking element may also be formed of composites of two or more materials such as those described above. The composite may comprise intimate mixtures of materials, or it may consist of a core material coated with a second or subsequent material placed on or around the core. A preferred embodiment of a composite material comprises a metal core coated with a plastics or elastomeric material. The advantages of composites are several: The core, being made of a high density material would ensure that the locking element moved freely in electrostatic fields, whereas the other material or materials, provided either as a coating or in admixture with the high density material, could impart other properties such as softness or resilience that would have the effect of producing less noise when the second locking element engages with adjacent components of the device. In an alternative to coating a second locking element, those skilled in the art will appreciate that a similar advantageous effect could be achieved if the locking element were made of a high density material (such as steel) and the components of the container adjacent the locking element and adapted to engage the locking element could be coated with, or formed of, materials having the aforementioned softness or resilience.

In some embodiments of the present invention, the second locking element may comprise one or more balls. The or each ball may be adapted to move in or on a profile formed in the housing of the container and cooperate with the first locking element and other adjacent components to provide a reliable locking mechanism. In such a case the profile is preferably in the form of a socket, in which the ball can roll freely under the influence of gravity and in response to movement of the container.

In some embodiments, said profile may define a "home" position such that when the second locking element is disposed in said home position, said second locking element is disposed clear of and is thus not engageable with said first locking element, such that said cap may be removed from the housing. Said profile may be configured such that upon rotation or tilting of the container or device, said second locking element is displaced under gravity from its home position and is moved to a position in which it is engageable with or obstructs movement of said first locking element. Thus said profile may define an inclined surface that is configured to retain the second locking element in said home position when the container or housing is oriented in the first orientation (i.e. when the cap and housing are disposed in the same horizontal plane) but directs the second locking element into the path of the first locking element upon rotation or tilting of the container or device. Said inclined surface may therefore subtend an acute angle with said horizontal plane. Said container or device may define a longitudinal axis, and said inclined surface may define a plane that is inclined with respect to said horizontal plane and contains or is disposed substantially parallel to said longitudinal axis.

Said second locking element may be held captive on or in said profile within a region defined by one or more obstacles to said second locking element. Said obstacles may be configured to guide the second locking element to the home position when the container or device or oriented in said first orientation. When said second locking element is displaced from said home position to obstruct movement of said first locking element, said obstacles prevent movement of the second locking element outside said region. Thus if the container or device is not correctly held in said first orientation, said second locking element is displaced from the home position under gravity. Attempted movement of the cap causes the first locking element to engage the second locking element which is positioned to interfere with such movement. Continued movement of the cap may cause the first locking element to push the second locking element over said inclined surface until it abuts said one or more obstacles. Thereupon, further continued movement of the cap is prevented by the abutment of the second locking element on said obstacle(s) and interengagement of the first and second locking elements.

In some embodiments, said locking means may comprise two sets of first and second locking elements which are configured respectively to operate upon rotation (or tilting) of the container or device in opposite directions. Both sets may operate upon tilting (or rotation) of the container or housing. Thus each set may comprise a profile formed within the housing wherein the profile of one set slopes in an opposite direction from the profile of the other set.

The size of the second locking element may vary in accordance with considerations such as the overall size of the container and with the elements of the container surrounding the second locking element with which it must cooperate in order that the locking mechanism can function effectively. When the second locking element is in the form of a ball, it is preferred that it has a diameter of about 3mm to about 6mm, more particularly 4mm, although the skilled person will appreciate that the upper limit is generally not strictly critical and can be any size in proportion to the size of the device and the component parts with which it is intended to cooperate.

As referred to above, the second locking element may be formed of composite materials. Such a composite may comprise a core of a first material and a coating of a second. In a preferred embodiment, the second locking element is in the form of a ball that has a core of a first material and a coating of a second and different material. Still more preferably, the ball comprises a metal core coated in plastics or elastomeric material. Suitable coating materials may be chosen from any of the plastics and elastomeric materials mentioned hereinabove.

Containers of the present invention are generally formed of plastics materials, or other materials that are capable of holding electrostatic charge, for example insulating materials such as glass and ceramics. Containers formed entirely of these materials, or having components formed of these materials, particularly components that are adjacent the second locking element or that are intended to cooperate with it are particularly advantageously employed in the present invention.

The containers for use in the present invention could be any container that is used to store and dispense pharmaceutical preparations and for which a locking mechanism is particularly important to ensure that active substances are not improperly administered. Inhaler devices, in particular those of the type described in the aforementioned patent are particularly advantageously employed with the locking mechanism. However, the locking mechanism could be applied to any type of inhaler device such as active or passive devices, i.e. those that are respectively actuated by a compressed gas source or those that are actuated by the inspiration air flow generated by a patient. Suitable inhalers may be multiple dose or single dose devices, and they may contain active agent in a reservoir or in individual doses contained in capsules or blisters.

The amount of tilt or rotation that is required to actuate the gravity-actuated locking mechanism will depend on a number of factors such as the nature of the material or materials forming the second locking mechanism, the shape of the surface of the housing profile and also the contact surface between the second locking element and the housing profile. The skilled person can vary these factors to achieve the desired degree of sensitivity required of the locking mechanism. Preferably the angle of rotation or tilt needed to actuate the locking mechanism should not be so slight that the device is very sensitive to the least movement of the device by the user. For ease of handling and use, the container preferably will permit of slight rotation or tilt without actuating the locking mechanism. An angle of rotation or tilt in the order of at least about 5 degrees, more particularly at least about 5 to 15 degrees is acceptable. However, if a less sensitive locking mechanism is desirable a much higher tilt or rotation angle can be built into the locking mechanism, for example by adjusting the angle of said inclined surface as desired..

Any active substance useful in treating conditions of the lung, such as asthma or chronic obstructive pulmonary disease (COPD), or useful being administered through the lung to treat systemic disease states may be employed in containers of the present invention. Suitable active agents include: beta.2-adrenoreceptor agonists such, for example, as salbutamol, terbutaline, rimiterol, fenoterol, reproterol, adrenaline, dpirbuterol, isoprenaline, orciprenaline, bitolterol, salmeterol, formoterol, clenbuterol, procaterol, broxaterol, picumeterol, TA-2005, mabuterol and the like and their pharmacologically acceptable esters and salts; steroids, including any of the materials selected from the group consisting of budesonide, ciclesonide, mometasone, fluticasone, beclomethasone, flunisolide, loteprednol, triamcinolone, amiloride and rofleponide or a pharmaceutically acceptable salt or derivative of these active compounds, such, for example, as mometasone furoate, fluticasone dipropionate, beclomethasone dipropionate, triamcinolone acetonide or flunisolide acetate (where optically active, these materials can be used in the form of their active isomer or as an isomer mixture); anticholinergic bronchodilators such, for example, as ipratropium bromide and the like; anti-allergic medicaments such, for example, as sodium cromoglycate and nedocromil sodium; expectorants; mucolytics; antihistamines; cyclooxygenase inhibitors; leukotriene synthesis inhibitors; leukotriene antagonists, phospholipase-A2 (PLA2) inhibitors, platelet aggregating factor (PAF) antagonists and prophylactics of asthma; antiarrhythmic medicaments, tranquilisers, cardiac glycosides, hormones, antihypertensive medicaments, antidiabetic-, such for example as insulin, antiparasitic- and anticancer-medicaments, sedatives and analgesic medicaments, antibiotics, antirheumatic medicaments, immunotherapies, antifungal and antihypotension medicaments, vaccines, antiviral medicaments, vitamins, anti-oxidants, free-radical scavengers; COX II inhibitors such as celecoxib; NSAIDS; PDE4 inhibitors and PDE5 inhibitors; and proteins, polypeptides and peptides.

A number of proteins and peptides have a potential for being suitable for inhalation therapy and some of them are in various stages of development. Some examples are insulin, alpha-1-proteinase inhibitor, interleukin 1, parathyroid hormone, genotropin, colony stimulating factors, erythropoietin, interferons, calcitonin, factor VIII, alpha-1-antitrypsin, follicle stimulating hormones, LHRH agonist and IGF-I, Ketobemidone, Fentanyl, Buprenorfin, Hydromorfon, Ondansetron, Granisetron, Tropisetron, Scopolamin, Naratriptan, Zolmitriptan, Almotriptan, Dihydroergotamin, Somatropin, Calcitonin, Erythopoietin, Follicle stimulating hormone (FSH), Insulin, Interferons (alfa and beta), Parathyroid hormone, alfa-1-antitrypsin, LHRH agonists, vasopressin, vasopressin analoques, desmopressin, glucagon, corticotropin (ACTH), gonadotrophin (luteinizing hormone, or LHRH), calcitonin, C-peptide of insulin, parathyroid hormone (PTH), human growth hormone (hGH), growth hormone (HG), growth hormone releasing hormone (GHRH), oxytocin, corticotropin releasing hormone (CRH), somatostatin analogs, gonadotropin agonist analogs (GnRHa), human gatrial natriuretic peptide (hANP) recombinant human thyroxine releasing hormone (TRHrh), follicle stimulating hormone (FSH), and prolactin.

Other possible polypeptides include growth factors, interleukins, polypeptide vaccines, enzymes, endorphins, glycoproteins, lipoproteins, and polypeptides involved in the blood coagulation cascade, that exert their pharmacological effect systemically.

Following is a description by way of example only with reference to the accompanying drawings of embodiments of the present invention. The detailed structure of these embodiments, their construction and operation are described in detail in US 6,182,655. The following discussion and drawings therefore describe details of the gravity-actuated locking mechanisms and any other parts of the devices that need to be discussed in order to understand such locking mechanisms.

In the drawings:
Figure 1 is a graph illustrating the empirical relationship between the density of the second locking element needed to move freely on or within the housing profile within an electrostatic field and field strength of said field.
Figures 2a, 2b, and 2c show a device in accordance with the present invention in perspective view showing the device in various stages of cap removal.
Figure 3 shows the sledge or carriage that is connected to the cap and which contains the first locking element.
Figures 4a and 4b show a plan and perspective views of the lower portion of the housing.
Figures 5a and 5b show perspective views of part sections of a part of the device of Figure 2a along the axis B-B'.
Figure 6 is a section of the lower hemisphere showing the movement of the ball to its locking position as a result of the device being rotated about the A-A' axis.
Figure 7 is a section of the lower hemisphere showing the movement of the balls in response to the device being tilted out of the A-A' axis.

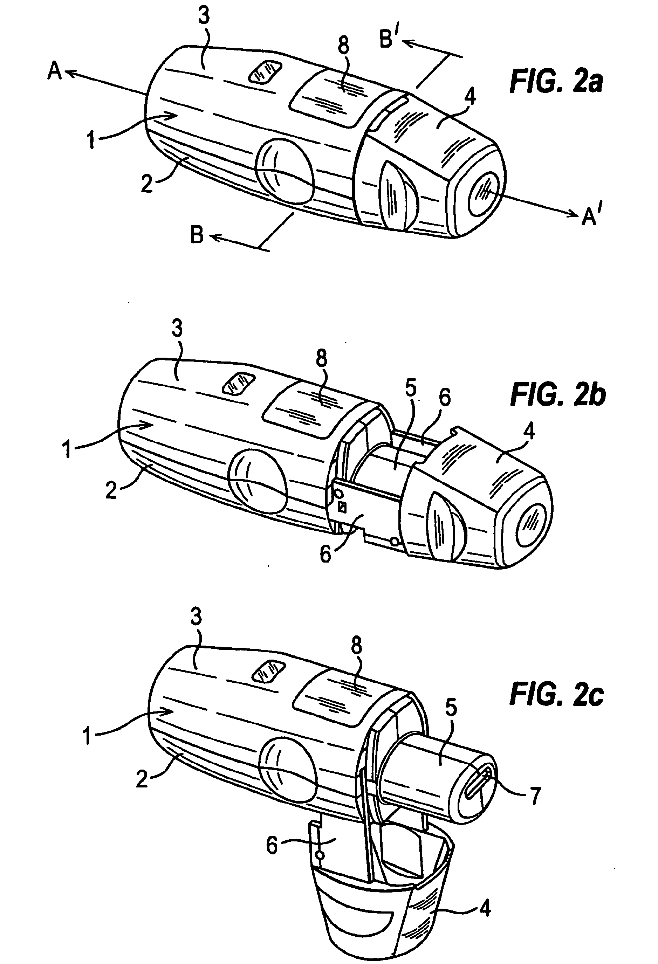

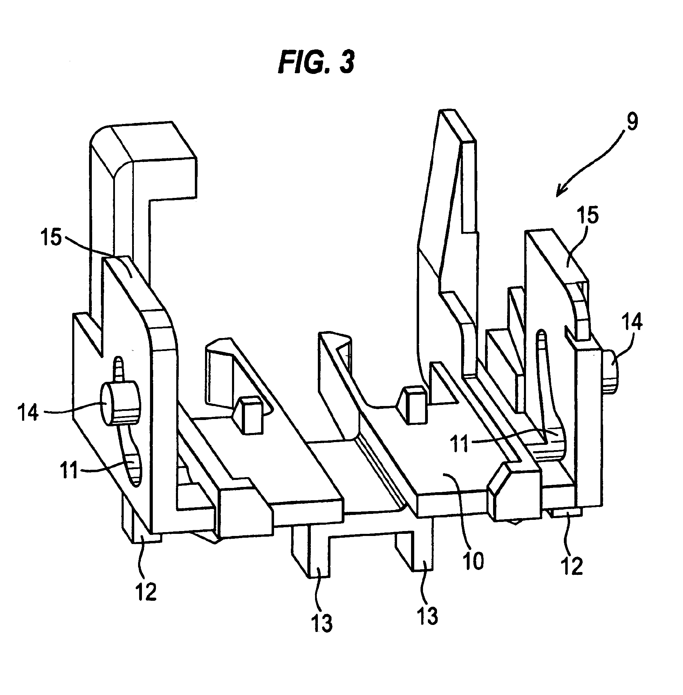

With reference to figures 2a, b and c, a device according to the present invention comprises an elongate housing (1) consisting of a lower portion (2) and an upper portion (3), and a cap (4) connected to the housing covering a mouth-piece (5). In Figure 2b the device is shown with the cap fully extended laterally with respect to the housing along the axis A-A', in a state of removal. In this position the mouth-piece (5) is partially visible although not yet accessible to a user. The cap has integral arms (6) that move along guide rails internal of the housing (not shown) and which are connected to a sledge containing first locking means (see Figure 3). Figure 2c shows the device with the cap fully extended and turned through ninety degrees to fully expose the mouth-piece to a user and ready a dose of active agent in the mouth-piece for inhalation. The movement of the cap is communicated through the integral arms (6) to a dosing mechanism internal of the housing (not shown) such that with the cap in the position shown in Figure 2c, a unit dose of drug substance is delivered into a dosing chamber ready to be administered to a patient when the device is actuated by a patient sucking on the mouth-piece through the hole (7). The device is shown in a substantially horizontal aspect, that is, it is neither substantially tilted relative to the elongate axis A-A', nor is it substantially rotated about the A-A' axis. In this orientation, a reservoir located internally of the housing and the top surface of which is shown (8), is located substantially vertically above the dosing mechanism (not shown) such that a unit dose of drug substance can be delivered by gravity to the dosing mechanism through an exit port located on the bottom surface of the reservoir. At the same time, in this orientation, a locking mechanism within the device and which will be described hereinunder in greater detail is in an unlocked position permitting removal of the cap enabling a user to actuate the device and thereby receive a correct dose of drug substance.

Figure 3 shows a perspective view of the sledge (9). Essentially the sledge consists of a body (10) having opposed side walls (15) and formed on each of said side walls a slot (11) and projection (14). The projections engage with slots provided in the cap arms (6) to connect the sledge with cap, and at the same time the slots (11) engage with projections provided on the arms (6). Through the cooperating slots and projections the movement of the cap as it is pulled open is communicated to the sledge, which is in this way able to move laterally in conformity with the cap. Once the cap has been extended fully it is then able to be swung through 90 degrees as shown in Figure 2c to permit a user access to the mouthpiece. On the bottom surface of the body (10) are provided projections (13) that engage guide rails provided in the lower portion of the housing (2) along which the sledge can run in a lateral direction. The body also contains abutment portions (12), which provided the first locking element of the gravity-actuated locking mechanism more fully described below.

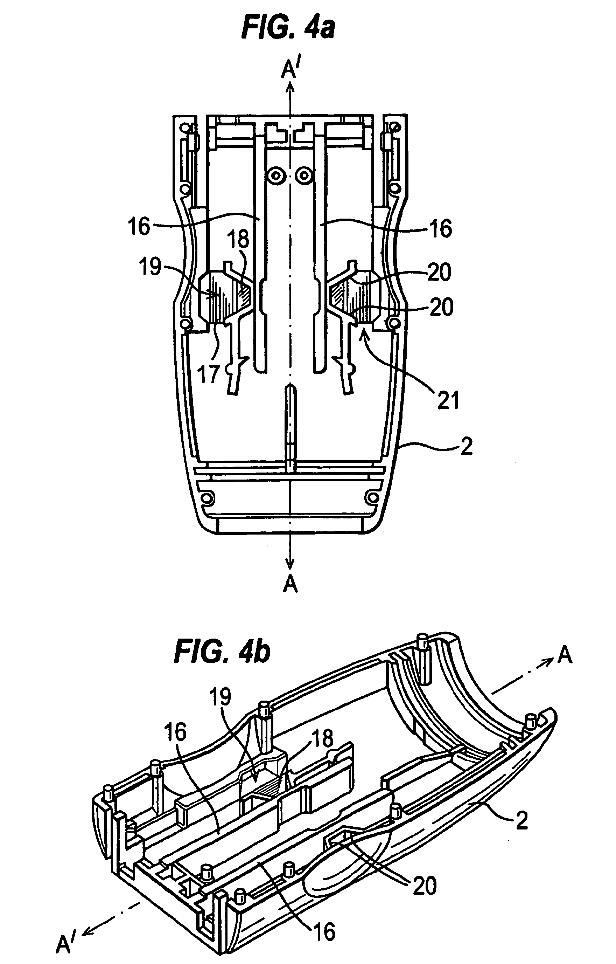

In Figure 4, the inner surface of the lower portion of the housing (2) contains a number of moulded features. In particular a housing profile (19) is moulded or pressed into the housing. This profile consists of an essentially flat surface (18) and a ramp (17) that extends upwards and outwards of the flat surface in the direction of the housing wall. Two angled walls (20) extend outward of the guide rails (16) in the direction of the housing wall but do not extend completely to the housing wall. A channel (21) is defined by the ends of the angled walls and the opposing housing wall. A blocking ball (not shown) sits in each of the flat surfaces (18) when the device is in an unlocked position and is retained in place by the walls (20) and the ramp (17).

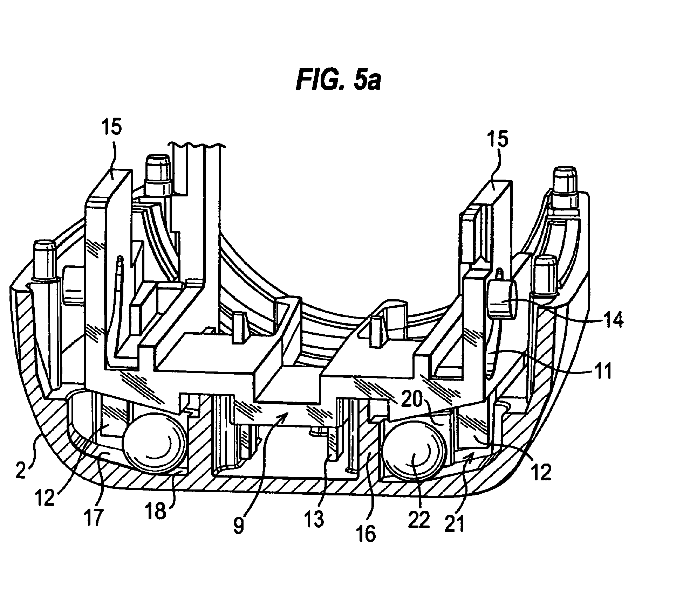

In Figure 5a, the sledge (9) is shown (not in section) mounted in the housing portion (2) on the guide rails (13). In such a mounted position the abutment portions (12) extend downwards towards the base of the housing (2) and are adapted to pass through the channels (21) when the sledge is moved laterally back and forth along the guide rails in conformity with the movement of the cap (4). Cap movement is communicated to the sledge by the cap arms (6) which are connected to the sledge by means of the projections (14) and the slots (11). For clarity, the arms (6) are not shown connected in this Figure 5a. In this Figure 5a, the sledge is in a fully retracted position representing the situation when the cap is closed. In this arrangement the abutment portions (12) do not extend into the channels (21). Locking balls (22) form a second locking element of the gravity-actuated locking mechanism and are shown in this figure in their unlocked positions such that if the cap was open causing the sledge to move the abutment portions would move through the channel unimpeded.

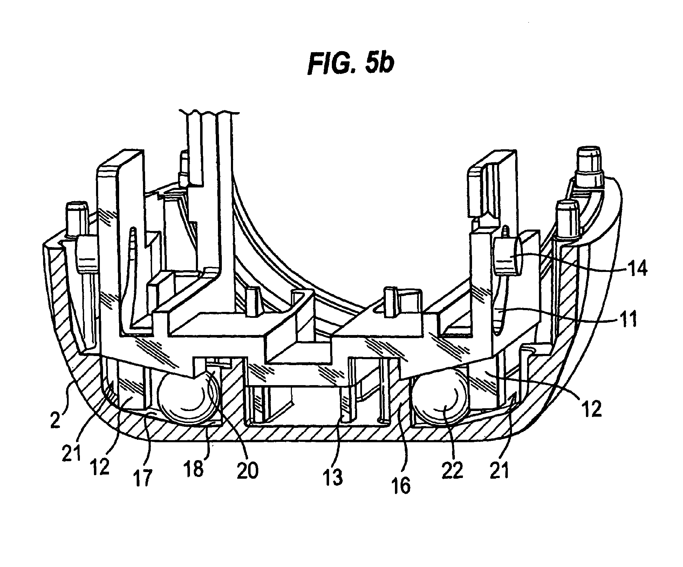

Figure 5b is substantially the same view as that shown in Figure 5a, only in this Figure the sledge (9) is drawn forward as a result of the cap (4) being drawn laterally outwards away from the housing (1) to assume the position shown in Figure 2b. In this Figure 5b, the abutment portions (12) can be seen as having advanced with the sledge through the channels (21), past the blocking balls (22). Again, in this Figure the blocking balls are in the unlocked position and the abutment portions (12) move past the balls unimpeded allowing the cap to open.

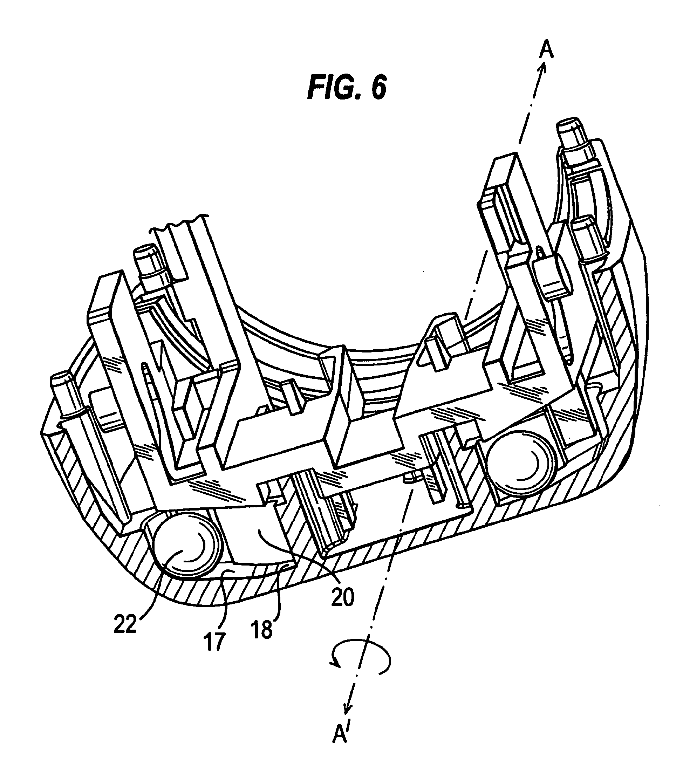

Figure 6 shows a view substantially the same as that of Figures 5. The device in this Figure 6 has been rotated about the axis A-A' in an anti-clockwise direction. The blocking ball (22) shown on the left of the Figure can be seen to move off the flat surface (18) and across the ramp (17) in the direction of the wall of the housing portion (2). The rotation must be sufficient for the blocking ball (22) to overcome the inertia of moving across the ramp (17). After reaching the critical point of rotation the ball moves under gravity into the position shown. Naturally, the skilled person will appreciate that the sensitivity of the locking mechanism can be adjusted by increasing or decreasing the inertial forces by either altering the mass of the blocking ball (22), or by altering the slope of the ramp (17), altering the contact angle between ball and housing profile or any combination of these. This movement occurs as a result of the rotation of the device. With the blocking ball (22) located on the ramp (17) any attempt to move the cap (4) and therefore the sledge (9) is impeded because the abutment portion (12) would come into contact with the blocking ball (22). In this manner, if a user has rotated the device out of the appropriate orientation for correct dosing the cap cannot be removed. It follows from the above that if the device is tilted in the clockwise direction, locking is effected by movement of the blocking ball (22) located on the right of the Figure into the path of the opposed abutment portion.

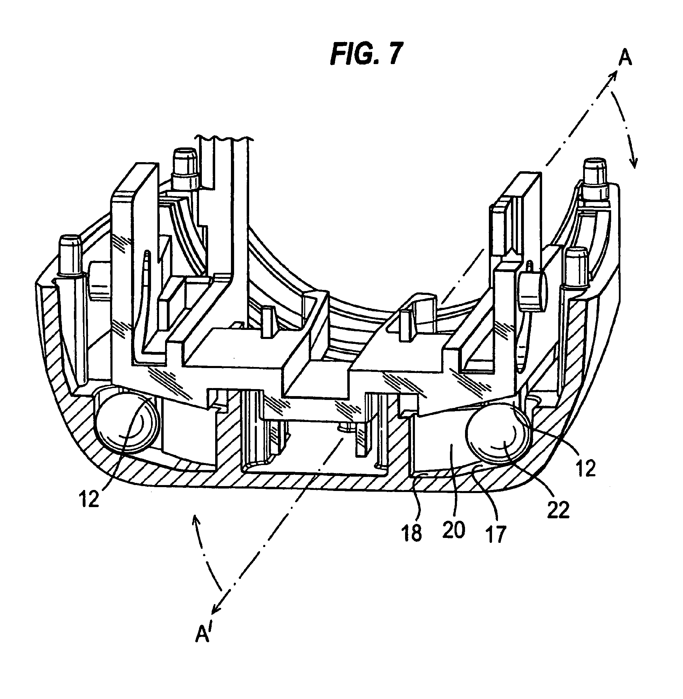

Figure 7 shows a view substantially the same as that of Figures 5 and Figure 6. In this Figure 7 however, the device has been tilted (as opposed to being rotated) out of the axis A-A' as if the cap end of the device was tilted upwards. In response to this tilting movement the blocking balls (22) both move across the ramp (17) guided by the angled walls (20). Once again, with the blocking balls (22) positioned on the ramp (17) in the channels (21), any attempt to move the cap (4) will result in the abutment portions (12) contacting the blocking balls (22) and actuate the locking mechanism. The sensitivity of the locking mechanism to the tilting motion of the device can be adjusted in the same manner as was discussed above in the case of the rotating motion.

## Claims

1. A container for dispensing an active agent, which container comprises a housing (1) containing said active agent, a cap (4) connected to the housing (1) and gravity-actuated locking means for locking together the housing (1) and the cap (4), said cap being in an unlocked state when the container is held in a first orientation, but when tilted or rotated out of said orientation said locking means are urged by gravity into locking engagement to lock the cap (4); wherein said locking means comprise a first locking element (9) that is connected to the cap (4) and is moveable in conformity with the cap (4) inside the housing (1) and a second locking element (22) that is moveable under force of gravity on a profile formed on the internal surface of the housing (19) in response to any tilting or rotating movement of the container out of the first orientation to engage the first locking element (9) in locking engagement; **characterised in that** the second locking element (22) is formed of at least one high density material and optionally one or more additional material, wherein the high density material has a density of at least about 1500kg/m³ which permits the second locking element (22) to move freely under gravity on the housing profile (19) even in the presence of an electrostatic field.

2. A container according to claim 1, wherein the cap (4) is its unlocked state when the container is held in an orientation such that both the housing (1) and the cap (4) are in the same horizontal plane, but when tilted or rotated out of said plane locking means (22) are urged by gravity into locking engagement to lock the cap (4).

3. A container according to claim 1 or claim 2, wherein the container is an inhaler device for dispensing a dose of active agent to the respiratory tract of a patient.

4. An inhaler device according to claim 3 further comprising:
the housing (1) having upper (3) and lower parts (2), an inner surface of the lower part having a housing profile (19) formed thereon;
a mouth-piece (5) attached or adapted to be attached to said housing;
the cap (4) connected to said housing and covering said mouth-piece (5), said housing (1) containing:
a reservoir (8) for storing an active agent having an exit port through which active agent can be released;
a dosing chamber for receiving a dose of active agent from the reservoir exit port; and
means for delivering the dose (7) from the dosing chamber to a patient via the mouth-piece (5) on a stream of gas;
the second locking element (22) located on the housing profile (19) and moveable on it from a first position to a second position under force of gravity in response to tilting or rotating movement of the device out of the first orientation to engage the first locking element (9) in locking engagement.

5. An inhaler device according to claim 4; wherein the cap (4) is in its unlocked state when the device is held in an orientation whereby the reservoir exit port is positioned vertically, or substantially vertically, over the dosing chamber.

6. A container or inhaler according to any of the preceding claims, wherein the second locking element is formed of a high density material and one or more additional material, wherein the one or more additional material is provided either as a coating on or around a core of the high density material or in admixture with the high density material.

7. A container or inhaler according to any of the preceding claims, wherein the high density material is selected from: high density plastics materials; metals such as steel or aluminium; glass or ceramics; or elastomeric materials such as natural rubber and nitrylbutadiene rubbers; preferably steel.

8. A container according to claim 7, wherein the at least one additional material is a plastics material or an elastomeric material.

9. A container or inhaler according to any of the preceding claims, wherein the one or more additional material is selected from polyoxymethylene (POM) or acrylonitrile butadiene styrene polymers (ABS).

10. A container according to any of the preceding claims, wherein the second locking element (22) is in the form of a ball.

11. A container according to any of the preceding claims, wherein the second locking element (22) is in the form of a steel ball coated with a plastics or elastomeric material.

12. A container according to claim 10 or claim 11, wherein the ball (22) has a diameter of 3 to 6 mm.

13. A container according to claim 12, wherein the ball (22) has a diameter of 4mm.

14. A container according to any preceding claim, wherein the second locking element (22) is formed of a material or materials having a density of at least about 1500kg/m³ to about 10,000kg/m³, preferably having a density of about 1500 to about 8000kg/m³.

## Patentansprüche

1. Behälter zum Verabreichen eines Wirkstoffes, wobei der Behälter ein Gehäuse (1), das den Wirkstoff enthält, einen Deckel (4), der mit dem Gehäuse (1) verbunden ist, und ein aufgrund der Schwerkraft betätigtes Verriegelungsmittel zum miteinander Verriegeln des Gehäuses und des Deckels (4) aufweist, wobei der Deckel in einem entriegelten Zustand ist, wenn der Behälter in einer ersten Ausrichtung gehalten wird, wenn er jedoch aus dieser Ausrichtung gekippt oder gedreht wird, das Verriegelungsmittel durch die Schwerkraft in einen Verriegelungseingriff zum Verriegeln des Deckels (4) gebracht wird, wobei das Verriegelungsmittel ein erstes Verriegelungselement (9), das mit dem Deckel (4) verbunden ist und in Übereinstimmung mit dem Deckel (4) innerhalb des Gehäuses (1) bewegbar ist, und ein zweites Verriegelungselement (22) aufweist, das Schwerkraftbedingt an einem an der Innerfläche des Gehäuses ausgebildeten Profil (19) entsprechend auf eine Kipp- oder Drehbewegung des Behälter aus der ersten Ausrichtung zum Ineingriffkommen mit dem ersten Verriegelungselement (9) bewegbar ist, **dadurch gekennzeichnet, dass** das zweite Verriegelungselement (22) zumindest aus einem Material mit hoher Dichte und optional aus einem oder mehreren zusätzlichen Materialien gebildet ist, wobei das Material mit hoher Dichte eine Dichte von wenigstens etwa 1500 kg/m³ hat, was dem zweiten Verriegelungselement (22) ermöglicht, sich unter Schwerkrafteinfluss auch dann auf dem Gehäuseprofil (19) frei zu bewegen, wenn ein elektrostatisches Feld vorhanden ist.

2. Behälter nach Anspruch 1, bei dem der Deckel (4) in seinem unverriegelten Zustand ist, wenn der Behälter in einer Ausrichtung gehalten wird, dass sowohl das Gehäuse (1) als auch der Deckel (4) in der gleichen horizontalen Ebene liegen, jedoch, wenn sie aus dieser Ebene gekippt oder gedreht werden, werden Verriegelungsmittel (22) unter Schwerkrafteinfluss in einen Verriegelungseingriff zum Verriegeln des Deckels (4) gebracht werden.

3. Behälter nach Anspruch 1 oder 2, bei dem der Behälter eine Inhalatorvorrichtung zum Verabreichen einer Wirkstoffdosis an die Atemwege eines Patienten ist.

4. Inhalatorvorrichtung nach Anspruch 3, ferner aufweisend:
einen oberen (3) und einen unteren Teil (2) des Gehäuses (1), wobei eine Innenfläche des unteren Teils ein darauf gebildetes Gehäuseprofil aufweist,
ein Mundstück (5), das an dem Gehäuse angebracht oder dazu geeignet ist, daran angebracht zu werden,
wobei der Deckel (4) mit dem Gehäuse verbunden ist und das Mundstück (5) bedeckt,
wobei das Gehäuse (1) beinhaltet:
ein Reservoir (8) zum Aufnehmen eines Wirkstoffes, das eine Auslassöffnung hat, durch welche der Wirkstoff abgegeben werden kann,
eine Dosierungskammer zum Empfangen einer Wirkstoffdosis von der Reservoirauslassöffnung, und
ein Mittel zum Zuführen der Dosis (7) von der Dosierungskammer zu einem Patienten über das Mundstück (5) in einem Gasstrom,
wobei das zweite Verriegelungselement (22) an dem Gehäuseprofil (19) angeordnet ist und auf diesem in Reaktion zu einer Kipp- oder Drehbewegung der Vorrichtung aus der ersten Ausrichtung zum Ineingriffkommen mit dem ersten Verriegelungselement von einer ersten Position zu einer zweiten Position unter Schwerkrafteinfluss bewegbar ist.

5. Inhalatorvorrichtung nach Anspruch 4, wobei der Deckel (4) in seinem entriegelten Zustand ist, wenn die Vorrichtung in einer Ausrichtung gehalten wird, bei der die Reservoirauslassöffnung vertikal oder im Wesentlichen vertikal über der Dosierungskammer angeordnet ist.

6. Behälter oder Inhalator nach einem der vorangegangenen Ansprüche, wobei das zweite Verriegelungselement aus einem Material mit hoher Dichte und einem oder mehreren Zusatzmaterialien gebildet ist, wobei das eine oder die mehreren zusätzlichen Materialien entweder als eine Beschichtung auf oder um einen Kern des Materials mit hoher Dichte herum oder als Beimischung zu dem Material mit hoher Dichte vorgesehen ist/sind.

7. Behälter oder Inhalator nach einem der vorangegangenen Ansprüche, bei dem das Material mit hoher Dichte gewählt ist aus: hochdichten Kunststoffmaterialien, Metallen wie Stahl oder Aluminium, Glas oder Keramik, oder elastomeren Materialien wie Kautschuk und Nitril-Butadien-Kautschuk, bevorzugt Stahl.

8. Behälter nach Anspruch 7, wobei das zumindest eine zusätzliche Material ein Kunststoffmaterial oder ein elastomeres Material ist.

9. Behälter oder Inhalator nach einem der vorangegangenen Ansprüche, wobei das eine oder mehrere zusätzliche Materialien aus Polyoxymethylen- (POM) oder Acrylnitril-Butadien-Styren- (ABS) Polymer gewählt ist.

10. Behälter nach einem der vorangegangenen Ansprüche, wobei das zweite Verriegelungselement (22) die Form einer Kugel hat.

11. Behälter nach einem der vorangegangenen Ansprüche, wobei das zweite Verriegelungselement (22) die Form einer Stahlkugel hat, der mit einem Kunststoff oder elastomerischen Material bedeckt.

12. Behälter nach Anspruch 10 oder 11, wobei die Kugel (22) einen Durchmesser von 3 bis 6 mm hat.

13. Behälter nach Anspruch 12, wobei die Kugel (22) einen Durchmesser von 4 mm hat.

14. Behälter nach einem der vorangegangenen Ansprüche, wobei das zweite Verriegelungselement (22) aus einem Material oder Materialien gebildet ist, die eine Dichte von wenigstens etwa 1500 kg/m³ bis etwa 10000 kg/m³, bevorzugter Weise eine Dichte von etwa 1500 bis etwa 8000 kg/m³ hat.

## Revendications

1. Conteneur pour distribuer un agent actif, lequel conteneur comprend un logement (1) contenant ledit agent actif, un capuchon (4) relié au logement (1) est un moyen de verrouillage actionné par gravité pour verrouiller ensemble le logement (1) et le capuchon (4), ledit capuchon étant dans un état non verrouillé lorsque le conteneur est tenu dans une première orientation, mais quand il est incliné ou tourné hors de ladite orientation, ledit moyen de verrouillage est poussé par gravité dans une mise en prise de verrouillage pour verrouiller le capuchon (4) ; dans lequel ledit moyen de verrouillage comprend un premier élément de verrouillage (3) qui est relié au capuchon (4) et est mobile conformément au capuchon (4) à l'intérieur du logement (1) et un second élément de verrouillage (22) qui est mobile par gravité sur un profil formé sur la surface interne du logement (19) en réponse à n'importe quel mouvement d'inclinaison ou de rotation du conteneur hors de la première orientation pour mettre en prise le premier élément de verrouillage (9) dans une mise en prise de verrouillage ; **caractérisé en ce que** le second élément de verrouillage (22) est formé d'au moins une matière de densité élevée et de manière facultative d'une ou plusieurs matières supplémentaires, dans lesquelles la matière de densité élevée présente une densité d'au moins environ 1 500 kg/m³ qui permet au second élément de verrouillage (22) de se déplacer librement sous la gravité sur le profil de logement (19) même en présence d'un champ électrostatique.

2. Conteneur selon la revendication 1, dans lequel le capuchon (4) est dans son état non verrouillé lorsque le conteneur est tenu dans une orientation telle que tant le logement (1) que le capuchon (4) sont dans le même plan horizontal, mais quand il est incliné ou tourné hors dudit plan, le moyen de verrouillage (22) est poussé par gravité dans une mise en prise de verrouillage pour verrouiller le capuchon (4).

3. Conteneur selon la revendication 1 ou la revendication 2, dans lequel le conteneur est un dispositif d'inhalation pour distribuer une dose d'agent actif aux voies respiratoires d'un patient.

4. Dispositif d'inhalation selon la revendication 3, comprenant en outre :
le logement (1) comportant des parties supérieure (3) et inférieure (2), une surface intérieure de la partie inférieure présentant un profil de logement (19) formé dessus ;
une embouchure (5) attachée ou conçue pour être attachée audit logement ;
le capuchon (4) relié audit logement et couvrant ladite embouchure (5), ledit logement (1) contenant :
un réservoir (8), pour stocker un agent actif, ayant un orifice de sortie à travers lequel un agent actif peut être libéré ;
une chambre de dosage pour recevoir une dose d'agent actif en provenance de l'orifice de sortie de réservoir ; et
un moyen pour délivrer la dose (7) depuis la chambre de dosage à un patient par l'intermédiaire de l'embouchure (5) via un jet de gaz ;
le second élément de verrouillage (22) situé sur le profil de logement (19) et mobile sur ce dernier d'une première position à une seconde position par gravité en réponse à un mouvement d'inclinaison ou de rotation du dispositif hors de la première orientation pour mettre en prise le premier élément de verrouillage (9) dans une mise en prise de verrouillage.

5. Dispositif d'inhalation selon la revendication 4 ; dans lequel le capuchon (4) est dans son état non verrouillé lorsque le dispositif est tenu dans une orientation de sorte que l'orifice de sortie de réservoir est positionné verticalement, ou sensiblement verticalement, au-dessus de la chambre de dosage.

6. Conteneur ou un inhalateur selon l'une quelconque des revendications précédentes, dans lequel le second élément de verrouillage est formé d'une matière de densité élevée et d'une ou plusieurs matières supplémentaires, 1 dans lesquelles la ou les plusieurs matières supplémentaires sont prévues soit comme un revêtement sur ou autour d'un noyau de la matière de densité élevée, soit en mélange avec la matière de densité élevée.

7. Conteneur ou un inhalateur selon l'une quelconque des revendications précédentes, dans lequel la matière de densité élevée est sélectionnée parmi : des matières plastiques de densité élevée ; des métaux comme l'acier ou l'aluminium ; du verre ou de la céramique ; ou des matières élastomères comme le caoutchouc naturel et les caoutchoucs nitrilebutadiène ; de préférence de l'acier.

8. Conteneur selon la revendication 7, dans lequel l'au moins une matière supplémentaire est une matière plastique ou une matière élastomère.

9. Conteneur ou inhalateur selon l'une quelconque des revendications précédentes, dans lequel la ou les plusieurs matières supplémentaires sont sélectionnées parmi le polyoxyméthylène (POM) ou des polymères d'acrylonitrile-butadiène-styrène (ABS),

10. Conteneur selon l'une quelconque des revendications précédentes, dans lequel le second élément de verrouillage (22) est sous la forme d'une bille.

11. Conteneur selon l'une quelconque des revendications précédentes, dans lequel le second élément de verrouillage (22) est sous la forme d'une bille d'acier recouverte d'une matière plastique ou élastomère.

12. Conteneur selon la revendication 10 ou la revendication 11, dans lequel la bille (22) présente un diamètre de 3 à 6 mm.

13. Conteneur selon la revendication 12, dans lequel la bille (22) présente un diamètre de 4 mm.

14. Conteneur selon l'une quelconque des revendications précédentes, dans lequel le second de verrouillage (22) est formé d'une matière ou de matières ayant une densité d'au moins environ 1 500 kg/m³ à environ 10 000 kg/m³, ayant de préférence une densité d'environ 1 500 à environ 8 000 kg/m³.
